# EUROPEAN PATENT APPLICATION

(11) **EP 1 872 806 A1**
(43) Date of publication of application: **02.01.2008**
(21) Application number: 06116209.5
(22) Date of filing: 28.06.2006
(51) Int. Cl.: A61L 27/44, A61L 27/54, A61L 27/10

(54) **Implant, its uses and methods for making it**

(71) Applicant: Vivoxid Oy, 20520 Turku (FI)
(72) Inventor: Kangasniemi, Ilkka, 20760, Piispanristi (FI); Peltola, Timo, 20540, Turku (FI); Salonen, Jukka, 20100, Turku (FI)
(74) Representative: Maskula, Silla Marjatta

(57) **Abstract**

The present invention relates to an implant comprising a source of oxygen capable of releasing oxygen and a material selected from the group consisting of biodegradable and/or bioactive glass, sol-gel produced silica and mixtures thereof. The invention also relates to the uses of the implant as well as to methods of making it.

## Description

The present invention relates to an implant useful in the prevention and/or treatment of infections and in promotion of tissue healing and/or regeneration. The invention relates also to uses of the said implant and to methods of making it.

### BACKGROUND OF THE INVENTION

Following publications, references and materials are used herein to illuminate the background of the invention, and they are incorporated by reference. In particular, the cases providing additional details respecting the practice are incorporated by reference.

Oxygen is a known antibacterial agent. It can be made use of in wound dressings, as has been disclosed in e.g. WO 2004/091675 presenting an oxygen releasing bandage based on a complex between polyvinyl acetate and hydrogen peroxide. Document WO 2004/075944 presents a medical device having a porous coating comprising hydrogen peroxide. The body of the medical device is made of a polymer and the porous coating is made of a polymer.

Bioactive glass is a known bioactive material. Unlike most other bioactive materials, it is easy to control the manufacturing properties of bioactive glass, the rate of its chemical reactions and the biological response caused by it by changing the chemical composition of bioactive glass itself. Bioactive glass has been used in different types of implants, such as bone fillers/substitutes, bone growth promoting materials, middle ear prostheses etc. Some compositions of bioactive glasses are known to have antimicrobial effects e.g. US 6,190,643 B1 and US 6,342,207.

The problem of many conventional implants is that they are prone to infections because they allow microbial growth on their surface at the implantation site. There are many medical conditions that require the removal of tissue, such as neoplastic tissue, infected or traumatized tissue, which needs to be replaced with a permanent or temporary therapeutic material until the defect is healed or the tissue regenerated. In severe cases, compromised neovascularization, i.e. the slow formation of a new capillary network, may be a problem. If the defect is large a significant shortage of oxygen, anoxia, restricting cell proliferation and tissue organization and, perhaps, causing death of additional cells may emerge in the center of the defect. Oxygen depletion may occur also at the tissue/implant interface, slowing down the initial processes of tissue healing.

### OBJECT AND GENERAL SUMMARY OF THE INVENTION

The object of the invention is to minimise or even eliminate the problems existing in the prior art.

One object of the present invention is an implant useful in preventing and/or managing/eradicating microbial infections.

Another object of the present invention is to manufacture an implant useful in treating or replacing diseased tissue.

Another object of the present invention is to manufacture an implant useful in enhancing the healing or regeneration of tissue, compromised due to delayed access to oxygen physiologically provided by the forming capillary network.

In order to achieve the above-mentioned objects the present invention is characterised in what is defined in the characterising parts of the independent claims presented hereafter.

Typical implant according to the present invention comprises a source of oxygen capable of releasing oxygen and a material selected from the group consisting of bioactive and/or biodegradable glass, sol-gel produced silica and mixtures thereof.

Typically the implant according to the present invention is for use in the treatment and/or prevention of infections, such as infected dental root canals, infected chronic cutaneous wounds and ostitis, such as osteomyelitis.

Furthermore, the implant according to the present invention is typically also for use in traumatology, dentistry, otorhinolaryngology, orthopedics, surgery and internal medicine.

Typically the implant according to the present invention is also for use in the promotion of tissue healing and/or regeneration.

Typically composition according to the present invention comprising a source of oxygen capable of releasing oxygen and a material selected from the group consisting of bioactive and/or biodegradable glass, sol-gel produced silica and mixtures thereof, is for use as a medicament or as a therapeutic device.

Typical use according to the invention of a composition comprising a source of oxygen capable of releasing oxygen and a material selected from the group consisting of bioactive and/or biodegradable glass, sol-gel produced silica and mixtures thereof, is for the manufacture of an implant for treating and/or preventing infections, such as chronic infections.

One typical method of producing an implant capable of releasing oxygen, comprises according to the invention the following steps:
- mixing a material selected from a group consisting of bioactive and/or biodegradable glass, so├gel produced silica or their mixture, the material comprising a source precursor with a chemical agent,
- allowing the source precursor in the material and the chemical agent to react for a determined period under appropriate chemical and physical conditions whereby at least part of the source precursor is transformed into source of oxygen capable of releasing oxygen,
- washing and drying the obtained product and forming it to an implant.

Another typical method of producing an implant capable of releasing oxygen, comprises according to the invention the following steps:
- mixing granulous material selected from a group consisting of bioactive and/or biodegradable glass, sol-gel produced silica or their mixture with a source of oxygen, and
- forming the obtained product to an implant.

Still another typical method of producing an implant capable of releasing oxygen, comprises according to the invention the following steps:
- mixing a source of oxygen to the matrix of a material selected from a group consisting of bioactive and/or biodegradable glass or sol-gel produced silica during the preparation process, and
- forming the obtained product to an implant.

### DEFINITIONS AND DETAILED DESCRIPTION OF THE INVENTION

The terms used in this application, if not otherwise defined, are those agreed on at the consensus conference on biomaterials in 1987 and 1992, see Williams, DF (ed.): Definitions in biomaterials: Proceedings of a consensus conference of the European Society for Biomaterials, Chester, England. March 3-5, 1986. Elsevier, Amsterdam 1987, and Williams DF, Black J, Doherty PJ. Second consensus conference on definitions in biomaterials. In: Doherty PJ, Williams RL, Williams DF, Lee AJ (eds). Biomaterial-Tissue Interfaces. Amsterdam: Elsevier, 1992.

In this application, by bioactive material is meant a material that has been designed to elicit or modulate biological activity. The term biodegradable in this context means that it is degradable upon prolonged implantation when inserted into mammalian body. By biomaterial is meant a material intended to interface with biological systems to evaluate, treat, augment or replace any tissue, organ or function of the body. By biocompatibility is meant the ability of a material used in a medical device to perform safely and adequately by causing an appropriate host response in a specific location. By resorption is meant reduction/disintegration of biomaterial because of cellular activity or simple dissolution. By composite is meant a material comprising at least two different constituents, for example an organic polymer and a ceramic material.

Implants in this context are meant to comprise any kind of implant used within the body, such as artificial organs and parts thereof, joint implants, internal/external fixation devices, devices used for reconstruction or replacement of bones and tissues, devices used for supporting and /or stimulation of tissue healing or regeneration, devices used for filling defects in bones and materials used as sealant or posts in the root canal of a tooth. Depending on the application and purpose of the implant materials, they are expected and designed to be biocompatible and exhibit either longevity or controlled degradability in the body. The optimal degradation rate is directly proportional to the renewal rate of the tissue. In the case of bone tissue, a considerable proportion of the implant is preferably degraded by 6 weeks in the tissue. In cases where physical support to the healing tissues is desirable the degradation rate might be several months or even several years. In some embodiments of the invention the degradation rate may even be nonexistent. Furthermore, the invention can be made use of in medical devices such as canules, catheters and stents.

Infection in this context comprises various infections within the body of a mammal, for example human. Infections may occur inside the body, subcutaneously or on the surface of the body. Infection may also occur in a wound or in a corresponding defect or lesion.

Accordingly, the present invention relates to an implant comprising a source of oxygen capable of releasing oxygen and a material selected from the group consisting of bioactive and/or biodegradable glass, sol-gel produced silica and mixtures thereof. The bioactive and/or biodegradable glass can be prepared either conventionally or by a sol-gel process. Conventionally prepared bioactive glass is produced by melting process.

Surprisingly, it has been found out that by incorporating or combining a source of oxygen, i.e. a chemical agent capable of releasing oxygen, to bioactive material comprising bioactive and/or biodegradable glasses it is possible to obtain an implant with an improved anti-microbial and tissue growth promoting effect. This beneficial effect is better than can be expected by evaluating the individual implant components separately, i.e. it appears that the ions released from the bioactive material and the active oxygen components released from the source of oxygen produce a synergetic effect, resulting in enhanced prevention of infections and/or rehabilitation of tissues. Furthermore, it is assumed that the silica network of the material provides an useful matrix to embrace and contain the chemical source of oxygen while it also allows the release of oxygen and associated reaction products through the matrix.

The implant according to the present invention has an improved effect in treatment and/or prevention of infections. The oxygen released from the implant effectively reduces, removes or even eliminates bacteria, especially anaerobic bacteria at the implantation site. Furthermore, the implant is capable of producing Ca(OH)₂ and reactive oxygen species (ROS) that are detrimental for microbes. Simultaneously, these reactions achieve a raise in pH at the area surrounding the implant surface. This pH raise has an impact on the solubility of the material. The amount of ions released from the material can be thus controlled and optimised. As these ions show also antimicrobial effects, a synergetic effect is obtained.

Furthermore, the implant according to the present invention improves the growth and regeneration of the tissues in which it is situated. Oxygen released from the implant may produce marked increase in the amount of O₂ dissolved in tissue fluid, which is believed to promote cell and tissue growth at the implant surface and its immediate vicinity, thus adding to the known osteoconductive and osteopromotive as well as soft tissue growth promoting effect of bioactive material. Dissolved oxygen promotes and/or advances the growth of the tissue cells near the implant, even if the capillary formation in the tissue would not be complete or totally effective near the implant. Also the ions, which are released from the material enhance and stimulate the capillary formation. As explained above, the dissolved oxygen source raises the pH and changes the solubility of the implant, and thus also the positive effect of the ions to the capillary formation becomes evident.

The release of oxygen from the implant is preferably slow enough for not to irritate the cells and tissues in contact with the implant or to interfere with the normal inflammatory cell response, e.g. macrophages, at the implantation site. In a physiological situation, the tissues typically remove about 4.6 ml O₂ from each 100 ml blood passing through them. Different sources of oxygen release oxygen at different rates. The release rate depends, among other things, on the molecular stability of the source of oxygen, i.e. the chemical agent used, in a given biological environment. For example, calcium peroxide releases oxygen as a function of pH. As the pH drops, the calcium peroxide becomes more soluble and generates progressively higher ratios of molecular oxygen and reactive oxygen species (ROS).

The metabolic activity of both eukaryotes and prokaryotes is known to reduce the pH of the environment where they grow and proliferate. The concentration of reactive oxygen species formed by the implant is thereby increased. Reactive oxygen species are byproduct of normal cellular respiration and specifically synthesized by phagocyte cells like neutrophils and macrophages. The implant may thus be considered as "intelligent" material: in the beginning when the number and/or metabolic activity of eukaryotes and prokaryotes is high and pH low, the implant is releasing more oxygen species. When the number of eukaryotes and prokaryotes decreases, the pH is correspondingly raised and the release of molecular oxygen and oxygen species decreases.

Possible reactive oxygen species include e.g. hydrogen peroxide, hydroxyl ions, hydroxyl radicals and oxygen radicals. In addition to the pH of the environment other factors that influence the oxygen release rate include e.g. the chemical composition, temperature and physical form of the implant. The present invention enables, for example, to select a bioactive and/or biodegradable glass composition that elicits an appropriate pH, which makes it possible to make adjustments of the release rate of the source of oxygen according to the relevant needs.

Bioactive and/or biodegradable glass and/or silica can function as a carrier material of said oxygen source. Said oxygen source can also be contained in another material, as will be discussed more in detail below. The carrier material can also be selected such that is slows down the release rate or diffusion of oxygen.

According to one embodiment of the invention the composition of the active material selected from the group comprising conventionally produced bioactive and/or biodegradable glass, bioactive glass and silica produced by the sol-gel process, or their mixtures, can be selected so that the active material is in itself an antimicrobial material. The implant is typically bacteriocidic but it can also be bacteriostatic.

Generally, no additives are needed. However, according to one embodiment the implant may also comprise different additives, such as antibiotics, growth factors, etc. in order to enhance the results of the treatment. According to one specific embodiment of the invention the implant comprising sol-gel produced silica comprises peptide growth factors. Also silver containing sol-gel produced silicas may be suited for the use in the present invention. Fbssible additives can be incorporated either during the manufacture of the material or they can be added in suitable form to granulated or powdered material or they can be impregnated to the surface of the ready-made implant.

According to one embodiment of the invention the material comprising a source of oxygen may also kill viruses, fungi or other infectious organisms. It has also by definition a destructive impact on growth of anaerobic bacteria. It may be concluded that the source of oxygen strengthens the antimicrobial/antimicrobial biofilm properties of the material in a synergetic manner.

The release of oxygen preferably begins immediately after implantation of the implant and continues for a prolonged period of time, typically for 1 week up to 8 months, more typically for at least 2 weeks and up to 6 months, with a slow release rate, which can be for example, <1µl O₂/h in dental root canal with corresponding contact area to the implant surface and <1ml O₂/h in healing tissues.

Typically, the oxygen releasing implants are designed so that they are compatible with their intended object, purpose and location of use in the body. For example, in the dental root canal the primary aim is to eradicate any bacterial infection in the complicated anatomical system including the eventual side canals and the dentinal tubules. In this case the implant is not normally exposed to excessive amounts of body fluids and the clearance of O₂ from the root canal is restricted and slow. Therefore, the release rate of O₂ from the implant is also preferably slow. On the other hand, implants used in locations where they are exposed to a lot of fluctuating fluid in healing and living tissues the release rate must be adapted to meet the requirements defined by the needs of cells and tissues. In these cases the release rate is still slow but higher than in the root canal case. It can be observed that the implant according to the present invention typically prevents or at least reduces, because of its bacteriocidity, the formation of a bacterial biofilm on its surface. The implant, however, allows the dissolving of O₂ in the surrounding body fluid, which is paramount for the growth of cells and tissues. It is important that the time span during which the implant releases O₂ can be adjusted to serve different preventive and/or therapeutic purposes extending from simple disinfecting to long-term replacement of organs or tissues.

According to one embodiment of the invention the source of oxygen is selected from the group consisting of hydrogen peroxide, urea peroxide, calcium peroxide, magnesium peroxide, sodium percarbonate, potassium monopersufate and mixtures thereof. The amount of the oxygen releasing material in the implant is typically 0.1 - 30 weight-%, more typically 0.2 - 20 weight-%, most typically 0.5 - 15 weight-% of the total weight of the ready-to-use implant. The amount of the oxygen releasing material can be chosen according to the end use of the implant or according to the used source of oxygen. In some embodiments the amount of the oxygen releasing material in the implant is low, in the range of 0.5 - 10 weight-%, more typically 1 - 7 weight-%, most typically 3 - 6 weight-% of the total weight of the ready-to-use implant. According to one embodiment of the invention an implant intended for treatment of root canal comprises, for example, preferably 5 weight-% of calcium peroxide. Generally speaking the disintegration of calcium peroxide is relatively slow process in conditions at issue, and release of oxygen is occurring at slow rate. Preferably calcium peroxide is situated inside the silica network of the implant, i.e. distributed relatively evenly within the implant, where it slowly disintegrates and simultaneously releases oxygen.

It is known that bioactive material, such as bioactive glass, reacts in body fluids, and forms calcium phosphate as reaction product. The calcium phosphate molecules are present on the material surface, within the silica network of the material as well as in the nearby tissue. With the present invention, this calcium phosphate can be partly replaced by calcium peroxide, which is capable of slow and adjustable release of oxygen from the material into its nearby vicinity.

According to an embodiment of the invention, said source of oxygen is obtained by subjecting a source precursor included in the material, such as calcium oxide, to a hydrogen peroxide treatment. It has been found out that if bioactive glass having calcium oxide (CaO) as a network modifier is treated with diluted hydrogen peroxide, at least part of said calcium oxide transforms into calcium peroxide. A part of said calcium oxide also transforms into calcium hydroxide. A similar transformation can be made in sol-gel produced CaO doped silica. In other words, the implant according to one embodiment of the present invention can be bioactive and/or biodegradable glass that has been treated so as to comprise calcium peroxide in its network. Calcium peroxide can then release oxygen ions when coming into contact with body fluids and/or tissue. The longer the reaction time, the deeper into the implant the formation of calcium peroxide advances. For example, when the reaction is allowed to proceed for 5 days, typically a layer of 100 - 150 µm is achieved. Another possible control variable is the used hydrogen peroxide concentration and the reaction temperature. The modified implant product obtained is highly stable. The hydrogen peroxide used is typically quite weak, having a concentration below 10 vo├%, usually from 4 to 7 vo├%.

Said treatment can be made for example by simply mixing a suitable bioactive and/or biodegradable glass or sol-gel produced and CaO doped silica with hydrogen peroxide, allowing it to react for a determined period of time, such as from 2 days to one week, washing and drying the obtained product. The reaction temperature is in the range of 2 - 10°C, preferably about 4°-C. By adjusting the reaction time it is possible to control the amount of formed calcium peroxide and its distribution within the silica network.

According to one specific embodiment granulates of bioactive glass and hydrogen peroxide were mixed together in proportion 3:1 and allowed to react for a week at the temperature ranging from 4 to 8 °C. The peroxide concentration was 4 vol-%.

According to another embodiment of the invention, said implant further comprises a biocompatible polymer. Said biocompatible polymer may also be biodegradable. The polymeric material may be selected from the group consisting of biocompatible polymers, such as derivatives of methacrylic acid, acrylic acid and vinylpyrrolidone, polyolefins, polyethylene oxide, polyethylene glycols, polyvinylalcohol, polylactones, polycarbonates, polyanhydrides, aliphatic polyesters, polyorthoesters, copolymers of the above mentioned, polymers and copolymers based on units derived from hydroxyacids and natural polymers, such as sugars, starch, cellulose and cellulose derivatives, polysaccharides, polypeptides and proteins.

The polymeric material may thus be either a biostable or a biodegradable material. The material can be porous or it can become porous during the use and/or when in contact with the tissue. Biostable polymers do not dissolve or react in contact with body fluids or tissue. Some suitable biostable polymers are derivatives of acrylic acid or methacrylic acid, such as methyl(methacrylate). Some suitable biodegradable polymers are homo- and copolymers of lactones and polycarbonates. The polymer may be a biodegradable and/or bioresorpable polymer and/or a biopolymer, preferably derived from hydroxyacid units, the most preferred polymeric material being poly(e-caprolactone-dl-lactide) copolymer. Mixtures of any of the above-mentioned polymers and their various forms may also be used. For embodiments intended for root canal also gutta-percha may be used.

According to one preferable embodiment of the present invention, the polymeric material is selected from the group consisting of polymers derived from hydroxy acid units, such as hydroxy acid, hydroxy acid derivative such as cyclic ester of a hydroxy acid (lactone), a cyclic carbonate, such as trimethyl carbonate, L-, D- and DL-lactic acids, L-, D- and DL-lactides and e-caprolactone. According to yet another embodiment, the polymeric material is poly(e-caprolactone-dl-lactide) copolymer. Also polylactide-co-glycolide (PLGA) or polylactide can be used.

The oxygen source can be incorporated in said implant in any known manner. It can be for example homogenously dispersed throughout the material of the implant, it can in itself make the implant or it can be in the form of a coating on the surface of the implant. The source of oxygen can be also encapsulated in silica gel or biodegradable polymer. The implant can moreover be manufactured for example by mixing a powder of bioactive and/or biodegradable glass with a powder of oxygen releasing material.

The sol-gel produced silica may be in the form of gel, xerogel, ceramic or the like. The sol-gel produced silica can be pure silica or it may typically comprise CaO and/or P₂O₅. Sol-gel produced silica may comprise 0.1 - 100 mol-% SiO₂. Typically the sol-gel produced silica comprises 40 - 60 mol % SiO₂, 5 - 10 mol % P₂O₅, and 35 - 50 mol % CaO.

As an additional component of the implants, it is also possible to use pure calcium phosphate CaP or tricalcium phosphate. Hydroxyl apatite, hydroxyapatite, hydroxycarbonated apatite are another possible material. Moreover, other bioactive ceramic materials or bioactive or biodegradable polymers may be used. Also hydrogels can be used as a carrier matrix for the oxygen source.

According to one embodiment of the invention, said bioactive glass has the following composition:
SiO₂ in an amount of 45 wt-%,
Na₂O in an amount of 24.5 wt-%,
CaO in an amount of 24.5 wt-%,
P₂O₅ in an amount of 6 wt-%.

According to one embodiment of the invention, said bioactive and/or biodegradable glass has the following composition:
SiO₂ in an amount of 40 - 70 wt-%,
Na₂O in an amount of 0 - 34 wt-%,
K₂O in an amount of X - Y wt-%,
MgO in an amount of 0 - 30 wt-%,
CaO in an amount of 0 - 30 wt-%,
B₂O₃ in an amount of 0- 4 wt-%,
P₂O₅ in an amount of 0-10 wt-%.

According to one embodiment of the invention, said bioactive glass has the following composition:
SiO₂ in an amount of 53 - 60 wt-%,
Na₂O in an amount of 0 - 34 wt-%,
K₂O in an amount of 1 - 20 wt-%,
MgO in an amount of 0 - 5 wt-%,
CaO in an amount of 5 - 25 wt-%,
B₂O₃ in an amount of 0- 4 wt-%,
P₂O₅ in an amount of 0,5 - 6 wt-%,
provided that
Na₂O + K₂O = 16 - 35 wt-%
K₂O + MgO = 5 - 20 wt-%, and
MgO + CaO = 10 - 25 wt-%.

This composition has been disclosed in WO 96/21628, the content of which is herein incorporated by reference.

According to another embodiment of the invention, the bioactive glass has the composition of
SiO₂ is 53 wt-%,
Na₂O is 23 wt-%,
CaO is 20 wt-% and
P₂O₅ is 4 wt-%.

According to yet another embodiment of the invention the bioactive glass has the composition of
SiO₂ is 51-56 wt-%,
Na₂O is 7-9 wt-%,
CaO is 21-23 wt-%,
K₂O is 10-12 wt-%,
MgO is 1-4 wt-%,
P₂O₅ is 0,5-1,5 wt-% and
B₂O₃ is 0-1 wt-%,
provided that the total amount of Na₂O and K₂O is 17-20 wt-% of the starting oxides. This composition has been disclosed in WO 2004/031086, the content of which is herein incorporated by reference.

According to one embodiment of the invention, the implant according to the present invention is intended for use in the treatment and/or prevention of chronic infections, preferably infections associated with necrosis and osteomyelitis.

In the case of osteomyelitis for example, the treatment is problematic since the lesion is characteristically ischaemic and after treatment/resection there are no more blood vessels to bring oxygen to the lesion site. The implant according to the present invention can thus be used to sustain the remaining cells until neovasculation is completed (re-growth of blood vessels). At the same time, the reaction products released from the oxygen source are acting as an antimicrobial agent at the surface of the implant, killing infectious cells synergically with the ions released from the material. The present implant thus solves the problem encountered with the prior art implants.

According to another embodiment of the invention, the implant according to the present invention is intended for use in traumatology, dentistry, otorhinolaryngology, orthopedics, surgery and internal medicine.

In dental applications, the implant can be used for example in endodontics, i.e. root canal treatments, periodontics and cariology. In orthopedics, the implant material may be used for example in revision surgery for implanted hip prostheses. In general surgery, the implant may be used in form of a dressing to prevent infection after surgery. In otorhinolaryngology, said implant can be designed for example to serve as a transtympanic membrane-tube. Furthermore, said implant can be used in nasal septum and frontal sinus to treat or prevent infection, as well as in any kind of catheters of long use span, stents and tubes, such as trachea tube. The implant may also be used to treat bone infections.

According to one embodiment of the invention, said non-specific and specific chronic infection is selected from the group consisting of microbial infections, including actinomycosis, infections associated with tissue necrosis and osteomyelitis.

The details and embodiments given above in connection with the implant also apply to the use according to the invention.

In this specification, except where the context requires otherwise, the words "comprise", "comprises" and "comprising" means "include", "includes" and "including", respectively. That is, when the invention is described or defined as comprising specified features, various embodiments of the same invention may also include additional features.

### EXPERIMENTAL PART

Experiments were made in order to determine the effect of adding calcium peroxide to bioactive glass.

### Experiment A

### Example 1

### 1.1 Preparation of calcium peroxide

CaO + H₂O₂ → CaO₂· 8 H₂O

568 mg of CaO was reacted with 70 ml of 30 % H₂O₂ in 430 ml of water. The ingredients were mixed, covered and kept occasionally agitated at 4 °C for seven days. The precipitate was collected by filtration, washed with distilled water and dried at room temperature. 1160 mg of slightly yellowish CaO₂ powder was obtained.

### 1.2 Preparation of the Test Composition

10 mg of CaO₂ powder obtained in step 1.1 was mixed with bioactive glass S53P4 having the composition of SiO₂ is 53 wt-% of the final total weight, Na₂O is 23 wt-% of the final total weight, CaO is 20 wt-% of the final total weight and P₂O₅ is 4 wt-% of the final total weight, and a particle size of less than 25 µm, to add up to 1 g of final Test Composition.

### 1.3 Procedure of Testing

The Effect of the Test Composition was evaluated in a suspension of bacteria Enterococcus faecalis A197A (clinical). Bacterial test suspension was prepared by thawing the bacteria and pipeting 10 µl of the suspension into a test tube with 5ml TSB, Tryptic Soy Broth from Bacto^{™}. The tube was kept at 37 ºC overnight. Next day the suspension was washed with 10 ml of physiological NaCl and centrifuged for 10 min/10 000 rpm. The supernatant was removed and 5 ml physiological NaCl was added. The mixture was vortexed. The concentration of bacterial suspension was adjusted by making use of spectrophotometer to density of 0.2 (A660). The suspension was diluted to 1:5 with physiological NaCl solution. 50 µl of this suspension was used for each experiment.

The test sample contained 50 mg of the Test Composition in the form of powder and 25 µl physiological NaCl solution. The bacteria and the test sample were mixed and then incubated under agitation for 30 minutes at 37 °-C. The reaction was stopped with 925 µl of physiological NaCl and the sample (1:1) was diluted with physiological NaCl (1:10, 1:100, and 1:1000). 10 µl of each of the samples was cultured on Tryptic Soy Agar from Difco^{™}. The cultures were incubated overnight at 37 ºC and the number of bacterial colonies, CFU-count, was counted from the plates on the following day. The results are given in Table 1, defined as Test Example 1 a.

The same procedure was repeated with two parallel samples, defined asTest Examples 1 b and 1 c.

### Example 2

Example 1 was repeated except that in step 1.2, 30 mg of CaO₂ powder was used. The results of three parallel experiments, Test Examples 2a, 2b and 2c, are given in Table 1.

### Example 3

Example 1 was repeated except that in step 1.2, 50 mg of CaO₂ powder was used. The results of three parallel experiments, Test Examples 3a, 3b and 3c, are given in Table 1.

### Negative Control Samples

75 µl of physiological NaCl was incubated over night at 37 °C. The results of three parallel experiments, Negative controls I, II, III, are given in Table 1.

### Positive Control Samples

50 mg of sterilised E-glass having particle size < 25 µm and manufactured by Ahlström Oy, Finland, was mixed with 25 µl of physiological NaCl and 50 µl of bacterial suspension and incubated over night at 37 °C. The bacterial samples were diluted to 1:100 with physiological NaCl. The results of three parallel experiments, Positive controls I, II, III, are given in Table 1.

Positive control sample procedure was repeated. The results of three parallel experiments, Positive controls Ia, IIa, IIIa, are given in Table 1.

### Comparative example 2

50 mg of bioactive glass of composition S53P4, which was also used in Example 1, manufactured by Vivoxid Oy, Turku, Finland and having particle size of < 25 µm, was mixed with 25 µl of physiological NaCl and 50 µl of bacterial suspension. The samples were diluted to 1:10 with physiological NaCl and incubated over night at 37 °C. The results of three parallel experiments, Comparative examples 2a, 2b and 2c, are given in Table 1.

**Table 1 Results of the experiments, CFU stands for colony forming unit.**

| **Example** | **CFU*100** | **Log. CFU** |
|---|---|---|
| Negative control I | No bacteria | No bacteria |
| Negative control II | No bacteria | No bacteria |
| Negative control III | No bacteria | No bacteria |
| Positive Control I | 11 700 | 6.07 |
| Positive Control II | 11 000 | 6.04 |
| Positive Control III | 7200 | 5.86 |
| Positive control Ia | 9400 | 5.97 |
| Positive control IIa | 9900 | 6.00 |
| Positive control IIIa | 11 200 | 6.05 |
| Test Example 1a | 270 | 4.43 |
| Test Example 1 b | 450 | 4.65 |
| Test Example 1c | 460 | 4.66 |
| Test Example 2a | 170 | 4.23 |
| Test Example 2b | 80 | 3.90 |
| Test Example 2c | 150 | 4.18 |
| Test Example 3a | 30 | 3.48 |
| Test Example 3b | 50 | 3.70 |
| Test Example 3c | 30 | 3.48 |
| Comparative example 2a | 470 | 4.67 |
| Comparative example 2b | 350 | 4.54 |
| Comparative example 2c | 500 | 4.70 |

As can be seen from table 1, Negative control gave no bacterial growth as there was no source of bacteria used.

The amount of bacterial colonies was calculated to non-diluted samples.

In Positive controls, E-glass was used, resulting in at most 11700 bacterial colonies.

In Test Example 1, the number of bacterial colonies was at most 460, in Test Example 2 at most 170 and in Test Example 3, at most 50. Calcium peroxide thus has an effect of increasing the antibacterial effect of the composition and an increase in the amount of calcium peroxide increases said antibacterial effect.

### Experiment B

Following materials were used in the experiment:
Bioactive glass of composition S53P4 manufactured by Vivoxid Oy, Turku, Finland; E-glass manufactured by Ahlström Oy; Finland; Tryptic Soy Broth (TSB) from Bacto^{™}; Tryptic Soy Agar (TSA) from Scharlau Chemie S.A.; Fysiological NaCl from Baxter, 75 % CaO₂ from Sigma-Aldrich.

Samples were tested in a suspension of bacteria *Enterococcus faecalis* A197A (clinical). The bacterial test suspension was prepared as described above in point 1.3.

50 mg E-glass in the mixture of 25 µl physiological NaCl and 50 µl bacterial suspension was used as positive control sample without incubation; 75 µl physiological NaCl was used as negative control sample.

Comparative sample 1 comprised a mixture of 50 mg E-glass having particle size < 45 µm and 1 weight-% of CaO₂ in the mixture of 25 µl physiological NaCl and 50 µl bacterial suspension. The sample was diluted to 1:1000 with physiological NaCl and incubated over night at 37 °C.

Comparative sample 2 comprised a mixture of 50 mg E-glass having particle size < 45 µm and 5 weight-% of CaO₂ in the mixture of 25 µl physiological NaCl and 50 µl bacterial suspension. The sample was diluted to 1:100 with physiological NaCl and incubated over night at 37 °C.

Comparative sample 3 comprised 50 mg bioactive glass having particle size < 45 µm in the mixture of 25 µl physiological NaCl and 50 µl bacterial suspension. The sample was diluted to 1:100 with physiological NaCl and incubated over night at 37 °C.

Test example 1 comprised a mixture of 50 mg bioactive glass having particle size < 45 µm and 1 weight-% of CaO₂ in the mixture of 25 µl physiological NaCl and 50 µl bacterial suspension. The sample was diluted to 1:100 with physiological NaCl and incubated over night at 37 °C.

Test example 2 comprised a mixture of 50 mg bioactive glass having particle size < 45 µm and 5 weight-% of CaO₂ in the mixture of 25 µl physiological NaCl and 50 µl bacterial suspension. The sample was undiluted and incubated over night at 37 °C.

All the samples were prepared and examined in triplicate.

Results of the Experiment B are shown in Table 2.

**Table 2 Results of the experiments, CFU stands for colony forming unit.**

| **Example** | **CFU*100** | **Log. CFU** |
|---|---|---|
| Negative control I | | No bacteria |
| Negative control II | | No bacteria |
| Negative control III | | No bacteria |
| Positive control I | 12 000 | 6.08 |
| Positive control II | 13 000 | 6.11 |
| Positive control III | 20 000 | 6.30 |
| Test Example 1a | 1800 | 5.26 |
| Test Example 1b | 1700 | 5.23 |
| Test Example 1c | 1100 | 5.04 |
| Test Example 2a | 3 | 2.48 |
| Test Example 2b | 8 | 2.90 |
| Test Example 2c | 5 | 2.70 |
| Comparative example 1a | 10 000 | 6.00 |
| Comparative example 1b | 11 000 | 6.04 |
| Comparative example 1c | 10 000 | 6.00 |
| Comparative example 2a | 1200 | 5.08 |
| Comparative example 2b | 1900 | 5.28 |
| Comparative example 2c | 2600 | 5.41 |
| Comparative example 3a | 2300 | 5.36 |
| Comparative example 3b | 2500 | 5.40 |
| Comparative example 3c | 3400 | 5.53 |

From the results it can be concluded that the bioactive glass itself shows a minor antibacterial effect, see comparative example 3. Also the addition of CaO₂ to a "normal" E-glass gives a minor antibacterial effect, see comparative examples 1, 2. However, the combination of bioactive glass and CaO₂ gives an antibacterial effect much better than the direct sum effect of the components.

### Experiment C

### Dentin Block Examples

### Preparation of the Dentin Blocks

Test blocks, height 4 - 5 mm, diameter 5 +/- 1 mm, were prepared from the bovine teeth, lower incisors, with a diamond bur. The root canals of the blocks were widened with ISO 023 round bur. The smear layer was removed from the blocks with an ultrasonic bath treatment comprising 15 min in 17% EDTA, 4 min in 5% NaOCl, and 60 min in distilled water. The blocks were treated in tryptic soy broth, TSB, in ultrasonic bath for 10 min and sterilized by autoclaving 121 °C, 20 min in TSB. The sterilized blocks were incubated in TSB at 37 °C over night to control the sterilization being successful, in which case TSB solution should remain clear.

The blocks were then infected with *Enterococcus faecalis* A197A (clinical) by adding a few colonies in TSB-solution with a sterile loop. The blocks were kept in infected broth for 7 days at 37 °C. Negative controls were kept in sterile broth for 7 days at 37 °C. At the end of the infection period the monoinfection purity of the infected broth was checked by colony morphology and gram-staining.

### Samples

The antimicrobial effect of bioactive glass mixtures were studied as a paste-like consistency, 2 g/ml of 0.9% NaCl. BAG stands for bioactive glass.

Comparative paste 1 comprised a mixture of bioactive glass having particle size < 25 µm; Test paste 1 comprised a mixture of bioactive glass having particle size < 25 µm and 5 weight-% of CaO₂; Comparative paste 2 comprised bioactive glass having particle size < 45 µm; Test paste 2 comprised a mixture of bioactive glass having particle size < 45 µm and 5 weight-% of CaO₂; Test paste 3 comprised a mixture of 50 mg bioactive glass having particle size < 45µm and 10 weight-% of CaO₂.

Ultracal XS, which is a known Ca(OH)₂ containing agent for killing bacteria found in infected root canals including *Enterococcus faecalis,* produced by Ultracal Products Inc. was used as comparative example. It was a ready paste-like material.

The bacterial samples were taken at time points 3 and/or 7 days. Three parallel samples were used for each time point and each paste. Also three parallel positive and negative controls, comprising only 0.9% NaCl, were included for each time point. The test was made on cell culture plates having 24 wells.

### Experiment Procedure

General method reference: Haapasalo M, Orstavik D. In vitro infection and disinfection of dentinal tubules. J Dent Res 1987;66:1375-9.

The test pastes were used immediately after preparation. The test paste was applied first at the bottom of the well, on which the tooth block was then placed. The root canal of the block was filled carefully with the aid of a probe and finally the whole block was covered with the paste. The control blocks were prepared in a similar way using 0.9% NaCl instead of the paste. The empty wells of the plate were filled with 0.9% NaCl to ensure the humidity of air being sufficiently high. Plates were sealed with paraffin and incubated at 37 °C for the chosen time periods.

After incubation period the test materials were removed from the blocks by rinsing with 0.9% NaCl and cleaning with a round bur ISO 023. The dentin samples were taken with burs ISO 025, 029 and 031. The burs and the material adhered to them were collected into test tubes containing 2 ml TSB. The samples were vortexed for 15-20 s and 100 µl of each sample was cultivated on tryptic soy agar, TSA, plate. Plates were incubated at 37 °C over night. The growth was viewed the next day. After the cultivation the rest of the samples were stored at 37 °C for five days and these enriched samples were then cultivated on TSA plates. The purity of the *Enterococcus faecalis* cultures was checked by gram-staining.

The results are presented in tables 3a and 3b.

**Table 3a. Results of the experiments after incubation of 3 days. Explanations: - = no growth; + = 1 - 9 colonies; ++ = 10 - 100 colonies; +++ = >100 colonies; ++++ = >1000 colonies. Numbers in parentheses indicate the exact number of the colonies.**

| Sample | bur size 025 = + 100 µm = into dentin | bur size 029 + 200 µm = into dentin | bur size 031 + 300 µm into dentin |
|---|---|---|---|
| Negative control I | - | - | - |
| Negative control II | - | - | - |
| Negative control III | - | - | - |
| Positive control I | ++++ | ++++ | ++++ |
| Positive control II | ++++ | ++++ | ++++ |
| Positive control III | ++++ | ++++ | ++++ |
| Comparative Sample I | - | ++ (29) | ++ (14) |
| Comparative Sample II | +(1) | + (1) | + (1) |
| Comparative Sample III | - | +(2) | ++ (46) |
| Comparative Paste 1 I | - | - | - |
| Comparative Paste 1 II | - | - | - |
| Comparative Paste 1 III | - | - | - |
| Comparative Paste 2 I | - | - | - |
| Comparative Paste 2 II | - | - | - |
| Comparative Paste 2 III | - | - | - |
| Test Paste 1 I | - | - | - |
| Test Paste 1 II | - | - | - |
| Test Paste 1 III | - | - | - |
| Test Paste 2 I | - | - | - |
| Test Paste 2 II | - | - | - |
| Test Paste 2 III | - | - | - |
| Test Paste 3 I | - | - | - |
| Test Paste 3 II | - | - | - |
| Test Paste 3 III | - | - | - |

**Table 3b. Results of the enriched samples after incubation of 3 days. Explanations: - = no growth; + = growth**

| Sample | bur size 025 = + 100 µm into dentin | bur size 029 = + 200 µm = into dentin | bur size 031 + 300 µm into dentin |
|---|---|---|---|
| Negative control I | - | - | - |
| Negative control II | - | - | - |
| Negative control III | - | - | - |
| Positive control I | + | + | + |
| Positive control II | + | + | + |
| Positive control III | + | + | + |
| Comparative Sample I | + | + | + |
| Comparative Sample II | + | + | + |
| Comparative Sample III | + | + | + |
| Comparative Paste 1 I | - | + | + |
| Comparative Paste 1 II 1 | - | - | - |
| Comparative Paste 1 III | - | - | + |
| Comparative Paste 2 I | - | - | + |
| Comparative Paste 2 II | - | - | - |
| Comparative Paste 2 III | - | - | - |
| Test Paste 1 I | - | - | - |
| Test Paste 1 II | - | - | + |
| Test Paste 1 III | - | - | - |
| Test Paste 2 I | - | - | - |
| Test Paste 2 II | - | - | - |
| Test Paste 2 III | - | - | + |
| Test Paste 3 I | - | - | - |
| Test Paste 3 II | - | - | - |
| Test Paste 3 III | - | - | - |

### Conclusions on basis of Tables 3a and 3b:

Both negative and positive control show expected results, i.e. no bacterial growth in the dentinal tubules for negative controls and for positive controls bacterial growth is found deep in the dentinal tubules.

Comparative sample shows that an increasing bacterial growth is present in the dentin tubules the deeper the sample is taken from.

Bioactive glass alone and combined with CaO₂ appear to be an efficient antibacterial agent. However, the results from the enriched samples show that the test pastes 1, 2 and 3 give slightly better antibacterial results over pure bioactive glass especially deeper in the dentin structure

When the particle diameter of bioactive glass was larger, comparativepaste 2 and test paste 2, the antibacterial efficacy is almost the same without CaO₂ and with 5% of it. However, by increasing the amount of CaO₂ up to 10%, test paste 3, a complete bactericidal effect was achieved.

The experiment shows that paste of bioactive glass in aqueous paste-like suspension is a better dentin disinfecting agent than the Ca(OH)₂ containing commercial product Ultracal and that the efficacy of bioactive glass can be strengthened by addition of CaO₂.

### Experiment D

### Preparation of spray dried microspheres

The used raw materials for sol preparation are 24.0 g HCl, 40.0 g H₂O, 52.1 g tetra ethylene orthosilicate (TEOS). 2.32 g CaO₂ powder is mixed with the ready made sol whereby a homogenous mixture is obtained. The mixture is spray dried and CaO₂ comprising SiO₂ microspheres are obtained as end product.

## Claims

1. An implant comprising
- a source of oxygen capable of releasing oxygen and
- a material selected from the group consisting of biodegradable and/or bioactive glass, sol-gel produced silica and mixtures thereof.

2. Implant according to claim 1, **characterised in that** said implant further comprises a biocompatible polymer.

3. Implant according to claim 1, **characterised in that** said source of oxygen is selected from the group consisting of hydrogen peroxide, urea peroxide, calcium peroxide, magnesium peroxide, sodium percarbonate, potassium monopersufate and mixtures thereof.

4. Implant according to claim 1, **characterised in that** said source of oxygen is obtained by subjecting a source precursor included in the material, such as calcium oxide, to a hydrogen peroxide treatment.

5. Implant according to claim 1, **characterised in that** the composition of said bioactive glass is
SiO₂ in an amount of 53 - 60 wt-%,
Na₂O in an amount of 0 - 34 wt-%,
K₂O in an amount of 1 - 20 wt-%,
MgO in an amount of 0 - 5 wt-%,
CaO in an amount of 5 - 25 wt-%,
B₂O₃ in an amount of 0- 4 wt-%,
P₂O₅ in an amount of 0,5 - 6 wt-%,
provided that
Na₂O + K₂O = 16 - 35 wt-%
K₂O + MgO =5-20 wt-%, and
MgO + CaO = 10 - 25 wt-%.

6. Implant according to claim 1, **characterised in that** the composition of said bioactive glass is
SiO₂ is 53 wt-%,
Na₂O is 23 wt-%
CaO is 20 wt-% and
P₂O₅ is 4 wt-%t.

7. Implant according to claim 1, **characterised in that** it comprises bacteriosidic or bacteriostatic agents, or different additives, such as antibiotics or growth factors.

8. Implant according to claim 1 for use in the treatment and/or prevention of infections, such as infected dental root canals, infected chronic cutaneous wounds and ostitis, such as osteomyelitis.

9. Implant according to claim 1 for use in traumatology, dentistry, otorhinolaryngology, orthopedics, surgery and internal medicine.

10. Implant according to claim 1 for use in the promotion of tissue healing and/or regeneration.

11. Composition comprising a source of oxygen capable of releasing oxygen and a material selected from the group consisting of biodegradable and/or bioactive glass, sol-gel produced silica gel and mixtures thereof, for use as a medicament or as a therapeutic device.

12. Use of a composition comprising a source of oxygen capable of releasing oxygen and a material selected from the group consisting of biodegradable and/or bioactive glass, sol-gel produced silica gel and mixtures thereof, for the manufacture of an implant for treating and/or preventing infections.

13. Use according to claim 12, **characterised in that** infection comprises infections in dental root canals, infections in chronic cutaneous wounds and, preferably infections associated with necrosis or ostitis, such as osteomyelitis.

14. Method of producing an implant capable of releasing oxygen, comprising the following steps:
- mixing a material selected from a group consisting of bioactive and/or biodegradable glass, sol-gel produced silica or their mixture, the material comprising a source precursor with a chemical agent,
- allowing the source precursor in the material and the chemical agent to react for a determined period under appropriate chemical and physical conditions whereby at least part of the source precursor is transformed into source of oxygen capable of releasing oxygen,
- washing and drying the obtained product and forming it to an implant.

15. Method according to claim 12, **characterised in that** the material is in form of granules, fibres, tubes, coatings or spheres.

16. Method of producing an implant capable of releasing oxygen, comprising the following steps:
- mixing granulous material selected from a group consisting of bioactive and/or biodegradable glass, sol-gel produced silica or their mixture with a source of oxygen, and
- forming the obtained product to an implant.

17. Method of producing an implant capable of releasing oxygen, comprising the following steps:
- mixing a source of oxygen to the matrix of a material selected from a group consisting of bioactive and/or biodegradable glass or sol-gel produced silica during the preparation process, and
- forming the obtained product to an implant.
